# FASCICULE DE BREVET EUROPEEN

(11) **EP 4 003 537 B1**
(45) Date de publication et mention de la délivrance du brevet: **27.12.2023**
(21) Numéro de dépôt: 20746251.6
(22) Date de dépôt: 30.06.2020
(51) Int. Cl.: A61Q 13/00, A61L 9/01, C11B 9/00

(54) **UTILISATION DANS UN DIFFUSEUR ÉLECTRIQUE DE PARFUM D'UNE COMPOSITION AQUEUSE DE PARFUM CONTENANT UN AGENT TENSIO-ACTIF ET UN AGENT CONSERVATEUR**
VERWENDUNG EINES ELEKTRISCHEN PARFÜMZERSTÄUBERS EINER WÄSSRIGEN PARFUMZUSAMMENSETZUNG MIT EINEM TENSID UND EINEM KONSERVIERUNGSSTOFF
USE IN AN ELECTRIC PERFUME DIFFUSER OF AN AQUEOUS PERFUME COMPOSITION CONTAINING A SURFACTANT AND A PRESERVATIVE

(30) Priorité: 22.07.2019 FR 1908306
(43) Date de publication de la demande: 01.06.2022
(73) Titulaire: Produits Berger, 27520 Grand Bourgtheroulde (FR)
(72) Inventeur: HÉRAMBERT, Céline, 76500 La Londe (FR); GERARD, Corinne, 27400 INCARVILLE (FR)
(74) Mandataire: Novagraaf Technologies
(86) Numéro de dépôt international: PCT/FR2020/051137
(87) Numéro de publication internationale: WO 2021/014063

(56) Documents cités:
- EP-A2- 2 158 895
- US-A1- 2012 189 490
- US-A1- 2014 093 471

## Description

La présente invention concerne de manière générale l'utilisation d'une composition parfumée à base de tensioactif et de conservateur, dans un diffuseur électrique de parfum fonctionnant avec une céramique piézoélectrique.

Généralement, ce type de diffuseur électrique de parfum est connu de l'homme de l'art et est déjà commercialisé. Il s'utilise typiquement à l'aide de petites fioles d'huiles essentielles que le consommateur achète séparément. Le consommateur remplit le diffuseur d'eau, puis y introduit quelques gouttes d'huiles essentielles. En fonctionnement, une brume se forme et l'air est parfumé. L'intensité du diffuseur électrique est réglée par l'utilisateur avec le risque qu'il incorpore trop d'huile essentielle, ce qui peut l'exposer à un danger ou à l'inverse qu'il soit déçu si la quantité introduite est trop faible. US2012189490A1 présente par exemple ce type de diffuseur de parfum muni d'une céramique piézoélectrique.

Pour résoudre cette difficulté, le Demandeur a mis au point une composition parfumée en solution aqueuse à base d'huile essentielle, et/ou de matières premières de parfum naturelles ou de synthèse, pouvant présenter notamment des vertus aromacologiques et étant directement prête à l'emploi. Une telle composition est destinée à être utilisée telle quelle dans un diffuseur électrique de parfum fonctionnant avec une céramique piézoélectrique.

Avec une telle composition, il est nécessaire d'utiliser un agent tensioactif, afin de rendre miscible l'huile essentielle et la phase aqueuse et ainsi former une émulsion stable thermodynamiquement.

Le tensio-actif est une espèce amphiphile c'est-à-dire ayant une partie hydrophile et une partie lipophile. Dans le cas d'un tensioactif anionique, la partie hydrophile est chargée négativement, tandis que dans le cas d'un tensioactif cationique, la partie hydrophile est chargée positivement. Si le tensioactif est de nature amphotère, la partie hydrophile est chargée positivement et négativement, de sorte que la charge globale de la molécule est nulle. Dans le cas de la présente invention, le tensioactif utilisé dans la composition parfumée est non ionique, c'est-à-dire que la molécule de tensioactif ne comporte aucune charge nette. Les tensioactifs non ioniques présentent les avantages d'être peu onéreux, renouvelables, biodégradables, faiblement toxique et avec un faible pouvoir moussant. Néanmoins, de même que les autres types de tensioactifs, ils présentent l'inconvénient d'être difficilement vaporisables à l'aide du système piézoélectrique.

Par conséquent, en fonctionnement, des résidus sur la céramique piézoélectrique sont inévitables et peuvent générer un encrassement ou une détérioration prématurée de la céramique. En outre, des retombées grasses sur le socle ou le plan sur lequel repose le diffuseur électrique sont également attendues, dues aux propriétés moussantes des tensio-actifs, lors de la vaporisation du parfum.

Pour s'affranchir des inconvénients précités, le demandeur a mis au point une composition de parfum destinée à être utilisée dans un diffuseur électrique fonctionnant avec une céramique piézoélectrique. Le choix du tensio-actif et son pourcentage devront être adaptés pour limiter les inconvénients décrits ci-dessus tout en garantissant les performances recherchées : Qualité olfactive, intensité olfactive et promesse aromacologique.

Plus particulièrement, la présente invention a pour objet l'utilisation d'une composition aqueuse parfumée, dans un diffuseur électrique de parfum fonctionnant avec une céramique piézoélectrique, caractérisée en ce que ladite composition aqueuse parfumée comprend 0,1% à 0,3% en poids par rapport au poids total de ladite composition de parfum d'une huile essentielle et/ou de matières premières de parfum naturelles ou de synthèse, 0,1 à 1 % en poids par rapport au poids total de ladite composition de parfum d'un agent tensioactif non ionique, et 0,01 à 0,1 % en poids par rapport au poids total de ladite composition de parfum d'un agent conservateur.

En utilisant une telle composition parfumée, le consommateur n'a pas besoin de régler l'intensité du parfum que doit diffuser le diffuseur électrique

De manière avantageuse, on pourra utiliser, dans le cadre de la présente invention, un polysorbate à titre d'agent tensioactif, et de préférence le monolaurate de polyoxyéthylène(20)sorbitan.

De manière avantageuse, on pourra utiliser, dans le cadre de la présente invention, à titre d'agent conservateur, un biocide, et de préférence le 1,2-benzisothiazlin-3-one en solution aqueuse à 20%, présent à raison de 0,1% en poids par rapport au poids total de ladite composition de parfum.

D'autres avantages et particularités de la présente invention résulteront des exemples suivants donnés à titre d'exemple non limitatifs.

### EXEMPLES

### Produits

formulations parfumées commercialisées par PRODUITS BERGER pour la gamme « diffuseur électrique » sous les dénominations commerciales : « *Pomme vanille* », « *Paris Chic* », et *« Caresse de coton » ;*
Agent tensioactif : monolaurate de polyoxyéthylène(20)sorbitan, commercialisé sous la dénomination commerciale Tween 20 ;
Agent conservateur : 1,2-benzisothiazlin-3-one en solution aqueuse à 20%, présent à raison de 0,1% en poids.

### Tests

On évalue olfactivement en situation réelle dans une pièce la qualité olfactive des différentes formulations utilisées dans un diffuseur électrique à céramique piézoélectrique, ainsi que le pourcentage résiduel de résidus sur la céramique après 5 à 7 heures de fonctionnement du diffuseur.

Les résultats des tests sont indiqués ci-après, dans le tableau 1 :

**Tableau 1 :**

| Formulation | % de parfum | % de tween 20 Solution testée | Qualité olfactive |
|---|---|---|---|
| Pomme Vanillé UC | 0,1 | 0,6 | OK |
| Paris Chic | 0,1, | 0,3 | OK |
| Caresse de coton | 0,1 | 0,2 | OK |

Le pourcentage de résidu de la formule sur la céramique est inférieur à 1% par rapport au poids total de la formule avant utilisation.

## Revendications

1. Utilisation d'une composition aqueuse parfumée, dans un diffuseur électrique de parfum fonctionnant avec une céramique piézoélectrique, **caractérisée en ce que** ladite composition aqueuse parfumée comprend 0,1% à 0,3% en poids par rapport au poids total de ladite composition de parfum d'une huile essentielle et/ou de matières premières de parfum naturelles ou de synthèse, 0,1 à 1 % en poids par rapport au poids total de ladite composition de parfum d'un agent tensioactif non ionique, et 0,01 à 0,1 % en poids par rapport au poids total de ladite composition de parfum d'un agent conservateur.

2. Utilisation selon la revendication 1, selon laquelle l'agent tensioactif est un polysorbate, et de préférence le monolaurate de polyoxyéthylène(20)sorbitan.

3. Utilisation selon la revendication 1, selon laquelle l'agent conservateur est un biocide, et de préférence le 1,2-benzisothiazlin-3-one en solution aqueuse à 20%, présent à raison de 0,1% en poids par rapport au poids total de ladite composition de parfum.

## Patentansprüche

1. Verwendung einer wässrigen Duftstoffzusammensetzung in einem elektrischen Duftstoffzerstäuber, der mit einem piezoelektrischen keramischen Element arbeitet, **dadurch gekennzeichnet, dass** die wässrige Duftstoffzusammensetzung zu 0,1 bis 0,3 Gew.-% bezogen auf das Gesamtgewicht der Duftstoffzusammensetzung ein etherisches Öl und/oder natürliche oder synthetische Duftstoffrohstoffe, zu 0,1 bis 1 Gew.-% bezogen auf das Gesamtgewicht der Duftstoffzusammensetzung ein nicht ionisches Tensid und zu 0,01 bis 0,1 Gew.-% bezogen auf das Gesamtgewicht der Duftstoffzusammensetzung ein Konservierungsmittel umfasst.

2. Verwendung nach Anspruch 1, wobei das Tensid ein Polysorbat ist, und vorzugsweise Polyoxyethylen(20)sorbitanmonolaurat.

3. Verwendung nach Anspruch 1, wobei das Konservierungsmittel ein Biozid ist, und vorzugsweise 1,2-Benzisothiazlin-3-on in einer 20 %igen wässrigen Lösung, das in einer Menge von 0,1 Gew.-% bezogen auf das Gesamtgewicht der Duftstoffzusammensetzung vorliegt.

## Claims

1. Use of a fragranced aqueous composition in an electric fragrance diffuser operating with a piezoelectric ceramic, **characterized in that** said fragranced aqueous composition comprises 0.1% to 0.3% by weight, based on the total weight of said fragrance composition, of an essential oil and/or of natural or synthetic fragrance raw materials, 0.1 to 1% by weight, based on the total weight of said fragrance composition, of a nonionic surfactant, and 0.01 to 0.1% by weight, based on the total weight of said fragrance composition, of a preservative.

2. Use according to claim 1, wherein the surfactant is a polysorbate, and preferably polyoxyethylene (20) sorbitan monolaurate.

3. Use according to claim 1, wherein the preservative is a biocide, and preferably 1,2-benzisothiazolin-3-one in a 20% aqueous solution, present in an amount of 0.1% by weight based on the total weight of said fragrance composition.
